# EUROPEAN PATENT APPLICATION

(11) **EP 4 603 048 A1**
(43) Date of publication of application: **20.08.2025**
(21) Application number: 23894261.9
(22) Date of filing: 29.09.2023
(51) Int. Cl.: A61B 34/35

(54) **SURGERY ASSISTANCE SYSTEM, SURGERY ASSISTANCE ROBOT, CONTROL METHOD FOR SURGERY ASSISTANCE SYSTEM, AND ROBOT SYSTEM**

(30) Priority: 25.11.2022 JP 2022188287
(71) Applicant: KAWASAKI JUKOGYO KABUSHIKI KAISHA, Kobe-shi, Hyogo 650-8670 (JP)
(72) Inventor: KOBAYASHI, Ayataka, Hyogo 650-8670 (JP)
(74) Representative: Hoffmann Eitle
(86) International application number: PCT/JP2023/035812
(87) International publication number: WO 2024/111249

(57) **Abstract**

A robotic surgical system (100) includes a robot (60) including an encoder (E1) to detect an axis value indicating a drive amount of a drive axis, and operable to move a surgical instrument (4), a controller (31), and a first display (331). The controller is configured or programmed to, when a difference between a current axis value of the drive axis based on detection of the encoder and a past axis value of the drive axis based on detection of the encoder at a time at which the robot was stopped or a command value to drive the robot is greater than a threshold, provide a notification regarding an error and cause the first display to display the current axis value of the drive axis and a comparison value comparable to the current axis value of the drive axis.

## Description

### Technical Field

The present disclosure relates to a robotic surgical system, a surgical robot, a control method for a robotic surgical system, and a robot system.

### Background Art

Conventionally, a robotic surgical system is known. For example, Japanese Translation of PCT International Application Publication No. 2017-513551 discloses a robotic surgical system including a robot that includes a plurality of drive axes to move a surgical instrument, and a controller that controls driving of the robot. In the robotic surgical system, the controller compares the rotational position of a drive when the drive is stopped with the rotational position of the drive when the drive is restarted after being stopped, and performs a control to restart driving when the difference is within the tolerance, and not to restart driving when the difference is greater than the tolerance.

### Prior Art

### Patent Document

Patent Document 1: Japanese Translation of PCT International Application Publication No. 2017-513551

### Summary of the Invention

### Problems to be Solved by the Invention

In the robotic surgical system disclosed in Japanese Translation of PCT International Application Publication No. 2017-513551, the controller compares the rotational position of the drive when the drive is stopped with the rotational position of the drive when the drive is restarted after being stopped, and performs a control to restart driving when the difference is within the tolerance, and not to restart driving when the difference is greater than the tolerance. Therefore, when there is a difference greater than the tolerance between the rotational position of the drive when the drive is stopped and the rotational position of the drive when the drive is restarted after being stopped, and an error occurs, an operator cannot check the extent of the difference, and thus it is difficult for the operator to determine whether or not the robot can recover from the error.

The present disclosure is intended to solve the above problem. The present disclosure aims to provide a robotic surgical system, a surgical robot, and a control method for a robotic surgical system that each allow an operator to easily determine whether or not a robot can recover from an error when the error occurs in the drive amount of the robot.

### Means for Solving the Problems

In order to attain the aforementioned object, a robotic surgical system according to a first aspect of the present disclosure includes a robot including a plurality of drive axes and an encoder on each of the plurality of drive axes to detect an axis value indicating a drive amount of the drive axis, the robot being operable to move a surgical instrument, a controller, and a first display. The controller is configured or programmed to, when a difference between a current axis value of the drive axis based on detection of the encoder and a past axis value of the drive axis based on detection of the encoder at a time at which the robot was stopped or a command value to drive the robot is greater than a threshold, provide a notification regarding an error and cause the first display to display the current axis value of the drive axis and a comparison value comparable to the current axis value of the drive axis.

In the robotic surgical system according to the first aspect of the present disclosure, as described above, the controller is configured or programmed to, when the difference between the current axis value of the drive axis based on detection of the encoder, which indicates the drive amount of the drive axis, and the past axis value of the drive axis based on detection of the encoder at the time at which the robot was stopped or the command value to drive the robot is greater than the threshold, provide the notification regarding the error and cause the first display to display the current axis value of the drive axis and the comparison value comparable to the current axis value of the drive axis. Accordingly, when an error occurs because the difference between the current axis value of the drive axis and the past axis value of the drive axis or the command value is greater than the threshold, the current axis value of the drive axis and the comparison value comparable to the current axis value of the drive axis are displayed on the first display, and thus an operator can easily check how much the current axis value of the drive axis differs from the past axis value of the drive axis or the command value. Consequently, when an error occurs in the drive amount of the robot, the operator can easily determine whether or not the robot can recover from the error.

A surgical robot according to a second aspect of the present disclosure includes a robot body including a plurality of drive axes and an encoder on each of the plurality of drive axes to detect an axis value indicating a drive amount of the drive axis, the robot body being operable to move a surgical instrument, a controller, and a display. The controller is configured or programmed to, when a difference between a current axis value of the drive axis based on detection of the encoder and a past axis value of the drive axis based on detection of the encoder at a time at which the robot body was stopped or a command value to drive the robot body is greater than a threshold, provide a notification regarding an error and cause the display to display the current axis value of the drive axis and a comparison value comparable to the current axis value of the drive axis.

In the surgical robot according to the second aspect of the present disclosure, as described above, the controller is configured or programmed to, when the difference between the current axis value of the drive axis based on detection of the encoder, which indicates the drive amount of the drive axis, and the past axis value of the drive axis based on detection of the encoder at the time at which the robot was stopped or the command value to drive the robot body is greater than the threshold, provide the notification regarding the error and cause the first display to display the current axis value of the drive axis and the comparison value comparable to the current axis value of the drive axis. Accordingly, when an error occurs because the difference between the current axis value of the drive axis and the past axis value of the drive axis or the command value is greater than the threshold, the current axis value of the drive axis and the comparison value comparable to the current axis value of the drive axis are displayed on the first display, and thus an operator can easily check how much the current axis value of the drive axis differs from the past axis value of the drive axis or the command value. Consequently, it is possible to provide the surgical robot that allows the operator to easily determine whether or not the robot can recover from the error when the error occurs in the drive amount of the robot.

A control method for a robotic surgical system according to a third aspect of the present disclosure is a control method for a robotic surgical system, the robotic surgical system comprising a robot including a plurality of drive axes and an encoder on each of the plurality of drive axes to detect an axis value indicating a drive amount of the drive axis, the robot being operable to move a surgical instrument, and includes, when a difference between a current axis value of the drive axis based on detection of the encoder and a past axis value of the drive axis based on detection of the encoder at a time at which the robot was stopped or a command value to drive the robot is greater than a threshold, providing a notification regarding an error and causing a display to display the current axis value of the drive axis and a comparison value comparable to the current axis value of the drive axis.

In the control method for the robotic surgical system according to the third aspect of the present disclosure, as described above, the current axis value of the drive axis and the comparison value comparable to the current axis value of the drive axis indicating the drive amount of the drive axis are displayed on the display. Accordingly, when an error occurs because the difference between the current axis value of the drive axis and the past axis value of the drive axis or the command value is greater than the threshold, the current axis value of the drive axis and the comparison value comparable to the current axis value of the drive axis are displayed on the first display, and thus an operator can easily check how much the current axis value of the drive axis differs from the past axis value of the drive axis or the command value. Consequently, it is possible to provide the control method for the robotic surgical system that allows the operator to easily determine whether or not the robot can recover from the error when the error occurs in the drive amount of the robot.

### Advantageous Effect of the Invention

According to the present disclosure, the operator can easily determine whether or not the robot can recover from the error when the error occurs in the drive amount of the robot.

### Brief Description of the Drawings

FIG. 1 is a diagram showing the configuration of a robotic surgical system according to an embodiment.
FIG. 2 is a diagram showing the configuration of a medical manipulator according to the embodiment.
FIG. 3 is a diagram showing the configuration of a robot arm of the medical manipulator according to the embodiment.
FIG. 4 is a diagram showing an input of the medical manipulator according to the embodiment.
FIG. 5 is a block diagram showing the control configuration of the robotic surgical system according to the embodiment.
FIG. 6 is a diagram showing an example of a screen announcing the occurrence of a touch panel error according to the embodiment.
FIG. 7 is a diagram showing an example of a password entry screen for touch panel maintenance according to the embodiment.
FIG. 8 is a diagram showing an example of a selection screen for touch panel maintenance according to the embodiment.
FIG. 9 is a diagram showing an example of a screen prompting an operator to recover from an error during touch panel maintenance according to the embodiment.
FIG. 10 is a flowchart for illustrating an error notification process of a controller of a robotic surgical system according to a modified example of the embodiment. Modes for Carrying Out the Invention

An embodiment is hereinafter described on the basis of the drawings.

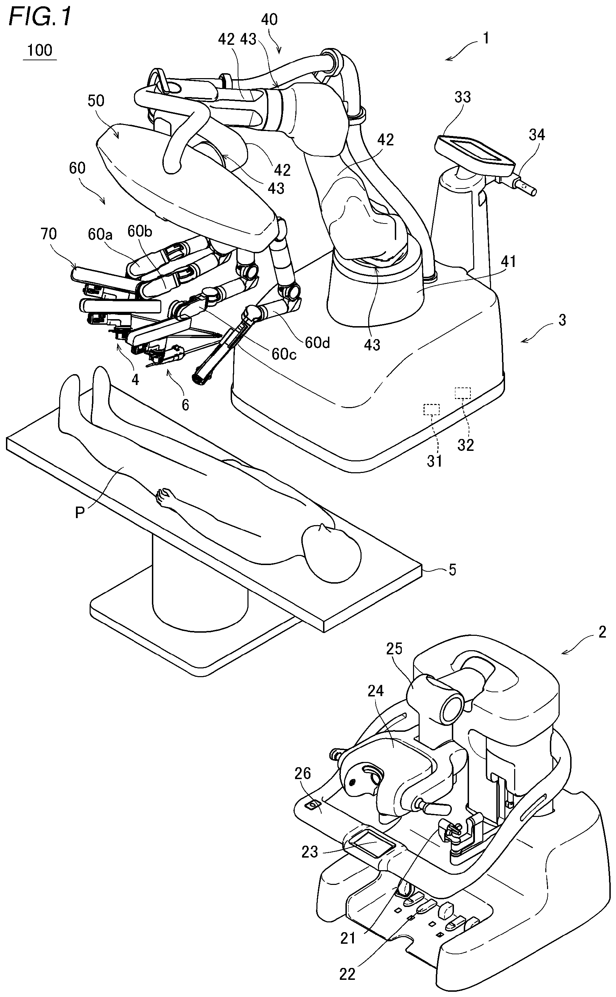

The configuration of a robotic surgical system 100 according to this embodiment is now described with reference to FIGS. 1 to 9. The robotic surgical system 100 includes a medical manipulator 1 that is a patientside apparatus and a remote control apparatus 2 that is an operator-side apparatus configured to operate the medical manipulator 1. The medical manipulator 1 includes a medical cart 3, and is configured to be movable. The remote control apparatus 2 is arranged apart from the medical manipulator 1, and the medical manipulator 1 is configured to be remotely operated by the remote control apparatus 2. An operator or a surgeon inputs a command to the remote control apparatus 2 to cause the medical manipulator 1 to perform a desired operation. The remote control apparatus 2 transmits the input command to the medical manipulator 1. The medical manipulator 1 operates based on the received command. The medical manipulator 1 is arranged in an operating room that is a sterilized sterile field. The medical manipulator 1 is an example of a "surgical robot" in the claims. The remote control apparatus 2 is an example of an "operator apparatus" in the claims. The robotic surgical system 100 is an example of a "robot system" in the claims.

The remote control apparatus 2 is arranged inside or outside the operating room, for example. The remote control apparatus 2 includes operation manipulator arms 21, operation pedals 22, a touch panel 23, a monitor 24, a support arm 25, and a support bar 26. The operation manipulator arms 21 include operation handles for the operator or the surgeon to input commands. Each of the operation manipulator arms 21 receives an operation amount for a surgical instrument 4. The monitor 24 is a scopetype display that displays an image captured by an endoscope 6. The support arm 25 supports the monitor 24 so as to align the height of the monitor 24 with the height of the face of the operator or the surgeon. The touch panel 23 is arranged on the support bar 26. The head of the operator or the surgeon is detected by a sensor (not shown) provided in the vicinity of the monitor 24 such that the medical manipulator 1 can be operated by the remote control apparatus 2. The operator or the surgeon operates the operation manipulator arms 21 and the operation pedals 22 while visually recognizing an affected area on the monitor 24. Command values are generated by operating the operation manipulator arms 21 and input to the remote control apparatus 2. The command values input to the remote control apparatus 2 are transmitted to the medical manipulator 1, and the medical manipulator 1 operates based on the command values. The operation manipulator arms 21 are examples of an "operation unit" in the claims. The touch panel 23 is an example of a "second display" in the claims.

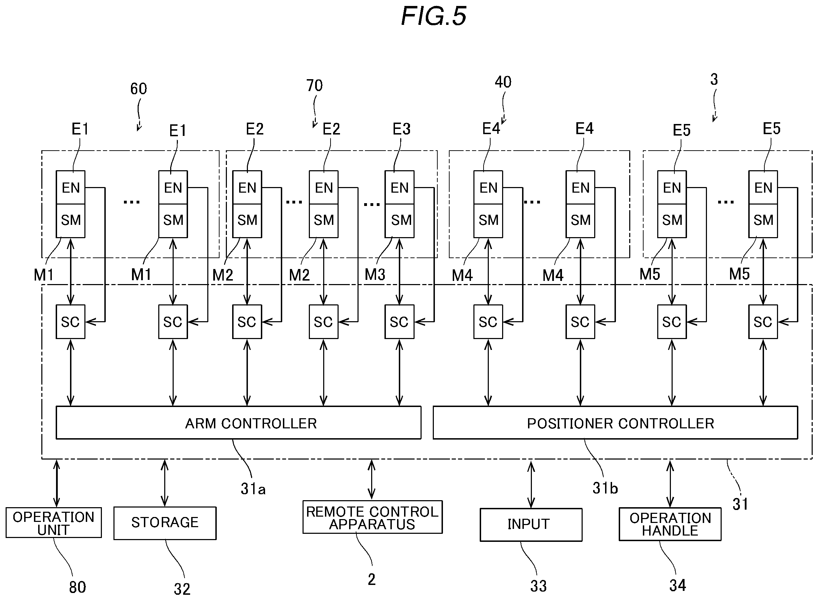

The medical cart 3 includes a controller 31 that controls the operation of the medical manipulator 1 and a storage 32 that stores programs or the like to control the operation of the medical manipulator 1. The controller 31 of the medical cart 3 controls the operation of the medical manipulator 1 based on the commands input to the remote control apparatus 2. As shown in FIG. 5, the controller 31 includes an arm controller 31a and a positioner controller 31b. The controller 31 is an example of the "controller" in the claims.

The medical cart 3 includes an input 33. The input 33 is configured to receive operations to move a positioner 40, an arm base 50, and a plurality of arms 60 or change their postures mainly in order to prepare for surgery before the surgery.

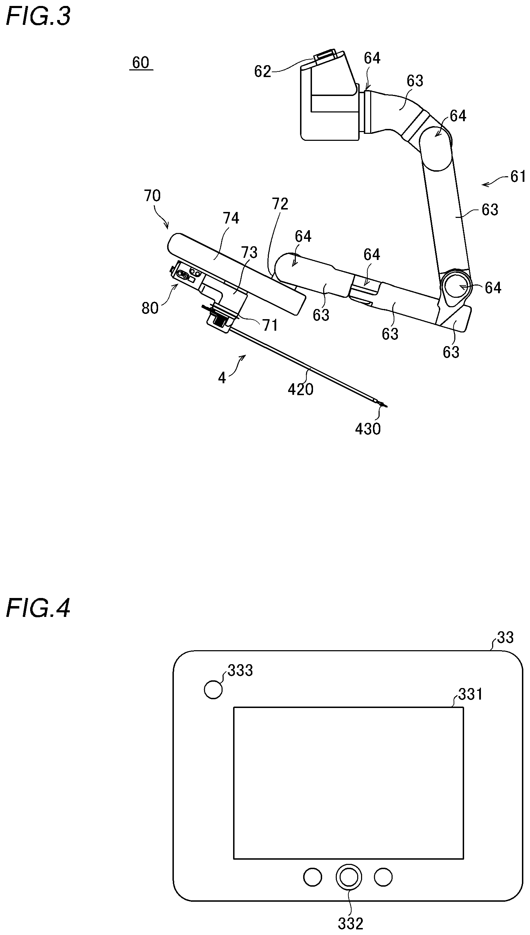

As shown in FIG. 4, a touch panel 331 is provided on the input 33 of the medical cart 3, and a joystick 332 is provided in the vicinity of the touch panel 331 to control movement of the positioner 40. An operation mode displayed on the touch panel 331 can be selected by a touch operation, and the positioner 40 can be operated three-dimensionally by operating the joystick 332. In addition, a reset button 333 is provided in the vicinity of the touch panel 331 to reset an error. The touch panel 331 is an example of a "first display" or a "display" in the claims.

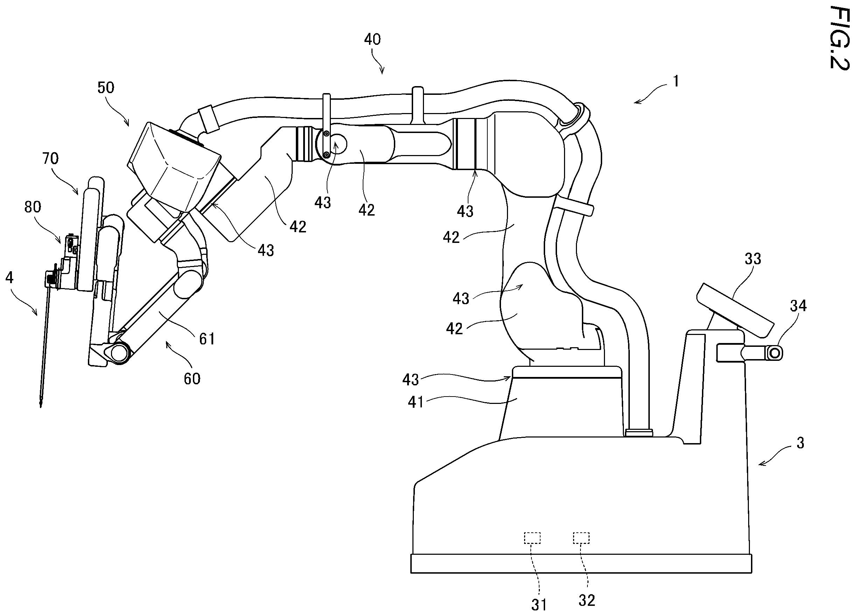

The medical manipulator 1 shown in FIGS. 1 and 2 is arranged in the operating room. The medical manipulator 1 includes the medical cart 3, the positioner 40, the arm base 50, and the plurality of arms 60. The arm base 50 is attached to a distal end of the positioner 40. The arm base 50 has a relatively elongated shape. The bases of the plurality of arms 60 are attached to the arm base 50. Each of the plurality of arms 60 is configured to be able to take a folded posture (stored posture). The arm base 50 and the plurality of arms 60 are covered with sterile drapes and used. The positioner 40 and the arm base 50 are examples of a "robot" or a "robot body" in the claims. The arms 60 are examples of a "robot", a "robot arm", or a "robot body" in the claims.

The positioner 40 includes a 7-axis articulated robot, for example. The positioner 40 is arranged on the medical cart 3. The positioner 40 moves the arm base 50. Specifically, the positioner 40 is configured to move the position of the arm base 50 three-dimensionally.

The positioner 40 includes a base 41 and a plurality of links 42 coupled to the base 41. The plurality of links 42 are coupled to each other by joints 43. The joints 43 are examples of a "drive axis" in the claims.

As shown in FIG. 1, the surgical instrument 4 is attached to a distal end of each of the plurality of arms 60. The surgical instrument 4 includes a replaceable instrument, the endoscope 6, etc., for example.

The configuration of the arms 60 is now described in detail.

As shown in FIG. 3, each of the arms 60 includes an arm portion 61 (a base 62, links 63, and joints 64) and a translation mechanism 70 provided at a distal end of the arm portion 61. The arms 60 are configured to three-dimensionally move the distal end sides with respect to the base sides (arm base 50) of the arms 60. The arms 60 have eight degrees of freedom. The plurality of arms 60 have the same configuration as each other. The joints 64 are examples of a "drive axis" in the claims.

The translation mechanism 70 is provided on the distal end side of the arm portion 61, and the surgical instrument 4 is attached to the translation mechanism 70. The translation mechanism 70 translates the surgical instrument 4 in a direction in which the surgical instrument 4 is inserted into a patient P. Furthermore, the translation mechanism 70 translates the surgical instrument 4 relative to the arm portion 61. Specifically, the translation mechanism 70 includes a holder 71 to hold the surgical instrument 4. Servomotors M2 (see FIG. 5) are housed in the holder 71. The servomotors M2 rotate rotors provided in the surgical instrument 4. The rotors of the surgical instrument 4 are rotated such that a pair of forceps is operated.

The arm portion 61 includes a 7-axis articulated robot arm. The arm portion 61 includes the base 62 configured to attach the arm portion 61 to the arm base 50, and a plurality of links 63 coupled to the base 62. The plurality of links 63 are coupled to each other by the joints 64.

The translation mechanism 70 is configured to translate the surgical instrument 4 attached to the holder 71 along a Y direction (a direction in which a shaft 420 extends) by translating the holder 71 along the Y direction. Specifically, the translation mechanism 70 includes a proximal end side link 72 connected to the distal end of the arm portion 61, a distal end side link 73, and a coupling link 74 provided between the proximal end side link 72 and the distal end side link 73. The holder 71 is provided on the distal end side link 73.

The coupling link 74 of the translation mechanism 70 is configured as a double speed mechanism that moves the distal end side link 73 relative to the proximal end side link 72 along the Y direction. The distal end side link 73 is moved along the Y direction relative to the proximal end side link 72 such that the surgical instrument 4 attached to the holder 71 is translated along the Y direction. The distal end of the arm portion 61 is connected to the proximal end side link 72 so as to rotate the proximal end side link 72 around an X direction perpendicular to the Y direction.

As shown in FIG. 1, the endoscope 6 is attached to one (the arm 60b, for example) of the plurality of arms 60, and the surgical instrument 4 other than the endoscope 6 is attached to each of the remaining arms 60 (arms 60a, 60c, and 60d, for example). Specifically, in surgery, the endoscope 6 is attached to one of the four arms 60, and surgical instruments 4 (such as a pair of forceps) other than the endoscope 6 are attached to the other three arms 60.

As shown in FIG. 3, the surgical instrument 4 is removably connected to the holder 71 of the arm 60 of the robotic surgical system 100. Specifically, the surgical instrument 4 is removably connected to the arm 60 via an adapter (not shown). The adapter is a drape adapter for sandwiching a sterilized drape (not shown) for covering the arm 60 between the holder 71 and the adaptor.

As shown in FIG. 5, the arm 60 includes a plurality of servomotors M1, encoders E1, and speed reducers (not shown) so as to correspond to a plurality of joints 64 of the arm portion 61. An encoder E1 is provided in each of the plurality of joints 64. The encoders E1 are provided to detect axis values indicating the drive amounts of the joints 64. Specifically, the encoders E1 detect the rotation angles of the servomotors M1. The axis values of the joints 64 are acquired based on the rotation angles of the servomotors M1 detected by the encoders E1. The speed reducers slow down rotation of the servomotors M1 to increase the torques.

As shown in FIG. 5, the translation mechanism 70 includes the servomotors M2 to rotate the rotors provided in a driven unit of the surgical instrument 4, a servomotor M3 to translationally move the surgical instrument 4, encoders E2 and E3, and speed reducers (not shown). The encoders E2 and E3 detect the rotation angles of the servomotors M2 and M3, respectively. The speed reducers slow down rotation of the servomotors M2 and M3 to increase the torques. The servomotors M1, M2, and M3 are controlled by the arm controller 31a.

The positioner 40 includes a plurality of servomotors M4, a plurality of encoders E4, and a plurality of speed reducers (not shown) so as to correspond to a plurality of joints 43 of the positioner 40. An encoder E4 is provided on each of the plurality of joints 43. The encoders E4 are provided to detect axis values indicating the drive amounts of the joints 43. Specifically, the encoders E4 detect the rotation angles of the servomotors M4. The axis values of the joints 43 are acquired based on the rotation angles of the servomotors M4 detected by the encoders E4. The speed reducers slow down rotation of the servomotors M4 to increase the torques.

The medical cart 3 includes servomotors M5 to drive a plurality of front wheels (not shown) of the medical cart 3, encoders E5, speed reducers (not shown), and brakes. The speed reducers slow down rotation of the servomotors M5 to increase the torques. A potentiometer is provided on an operation handle 34 of the medical cart 3, and the servomotors M5 of the front wheels are driven based on a rotation angle detected by the potentiometer according to the twist of a throttle of the operation handle 34. Rear wheels (not shown) of the medical cart 3 are of the dual wheel type, and the rear wheels are steered based on rightward-leftward rotation of an operation handle 34. The servomotors M4 and M5 are controlled by the positioner controller 31b.

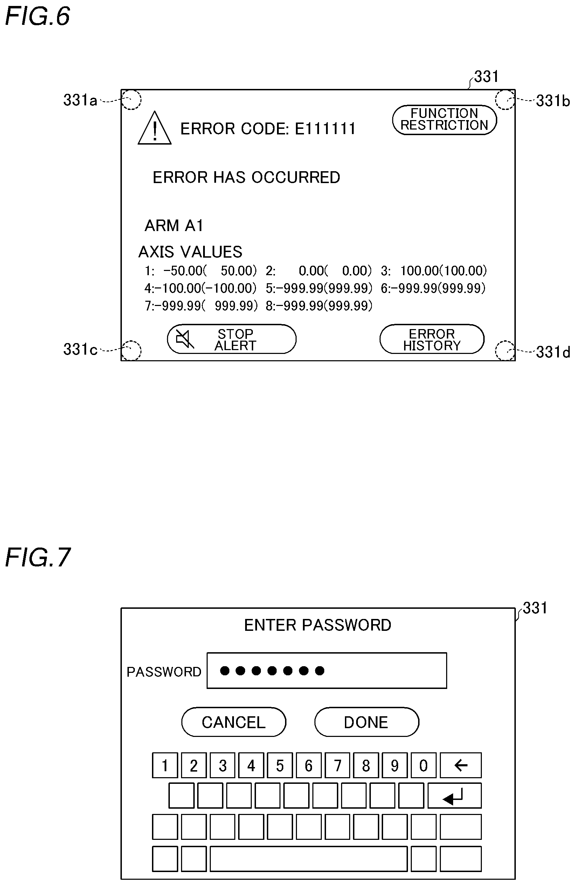

In this embodiment, as shown in FIG. 6 , when at the time of startup after shutdown, differences between the current axis values of the joints 64 (43) based on the detection of the encoders E1 (E4) and the past axis values of the joints 64 (43) based on the detection of the encoders E1 (E4) at the time at which the medical manipulator 1 was stopped at shutdown (past) are greater than a threshold, the controller 31 provides a notification regarding an error and causes the touch panel 331 to display the current axis values of the joints 64 (43) and comparison values comparable to the current axis values of the joints 64 (43).

When at the time of operation restart after operation interruption of the medical manipulator 1, differences between the current axis values of the joints 64 (43) based on the detection of the encoders E1 (E4) and command values at the time of operation interruption (past) are greater than a threshold, the controller 31 provides a notification regarding an error and causes the touch panel 331 to display the current axis values of the joints 64 (43) and the comparison values comparable to the current axis values of the joints 64 (43).

For example, as shown in an example of FIG. 6, a notification is provided on the touch panel 331 that an error has occurred in the drive amount (axis value) of an arm A1. The touch panel 331 also displays the current axis value of each joint 64 of the arm A1 and a comparison value in parentheses to the right of the axis value. In addition to error notification, an alert is issued by sound. A button for stopping the alert is displayed on the touch panel 331.

Thus, when an error occurs because the differences between the current axis values of the joints 64 (43) and the past axis values of the joints 64 (43) are greater than the threshold, the current axis values of the joints 64 (43) and the comparison values comparable to the current axis values of the joints 64 (43) are displayed on the touch panel 331, and thus an operator can easily check how much the current axis values of the joints 64 (43) differ from the past axis values of the joints 64 (43). Consequently, when an error occurs in the drive amount of the robot, the operator can easily determine whether or not the robot can recover from the error.

The comparison values displayed on the touch panel 331 together with the current axis values of the joints 64 (43) are, for example, the past axis values of the joints 64 (43), difference values between the past axis values and the current axis values of the joints 64 (43), command values for the past axis values, difference values between the command values for the past axis values and the current axis values, or predetermined reference values. The predetermined reference values are, for example, limit axis values or initial position axis values.

In other words, the controller 31 causes the touch panel 331 to display, as the comparison values, values based on at least one of the past axis values of the joints 64 (43), the difference values between the past axis values and the current axis values of the joints 64 (43), the command values for the past axis values, the difference values between the command values for the past axis values and the current axis values, or the predetermined reference values.

Thus, when the past axis values of the joints 64 (43) or the difference values between the past axis values and the current axis values of the joints 64 (43) are displayed as the comparison values, the current axis values of the joints 64 (43) can be easily compared with the past axis values of the joints 64 (43). When the command values for the past axis values or the difference values between the command values for the past axis values and the current axis values are displayed as the comparison values, the current axis values of the joints 64 (43) can be easily compared with the command values for the past axis values. When the predetermined reference values are displayed as the comparison values, it is possible to easily check how much the current axis values are compared to the limit values when the limit values that the axis values can take are displayed. Consequently, the operator can more easily determine whether or not it is possible to recover from the error.

When an error is detected based on the differences between the current axis values of the joints 64 (43) and the past axis values of the joints 64 (43), the axis values at the time of shutdown (past) of the medical manipulator 1 are compared with the axis values at the time of subsequent startup (current) of the medical manipulator 1, for example. When an error is detected based on the differences between the current axis values of the joints 64 (43) and the command values that generated the past axis values of the joints 64 (43), the command values at the time of operation interruption (past) of the medical manipulator 1 are compared with the axis values at the time of subsequent operation restart (current) of the medical manipulator 1, for example.

In other words, when the differences between the past axis values of the joints 64 (43) at a first time at which the medical manipulator 1 was shut down and the current axis values of the joints 64 (43) at a second time at which the medical manipulator 1 has been started up after the first time are greater than the threshold, the controller 31 provides a notification regarding the error and causes the touch panel 331 to display the current axis values of the joints 64 (43) at the second time and the comparison values.

Thus, when the robot moves due to an external force between shutdown and startup of the medical manipulator 1, the current axis values of the joints 64 (43) at the second time and the comparison values are displayed, and thus the operator can easily determine whether or not it is possible to recover from the error and start up or restart driving of the medical manipulator 1.

When an error is detected based on differences between the current axis values of the joints 64 (43) and command values to drive the arm 60, command values generated based on command inputs from the operator or the surgeon via the operation manipulator arm 21 are compared with the current axis values of the joints 64 based on the detection of the encoders E1. In other words, when the arm 60 is being driven in real time, command values based on the operation are compared with the actual drive amounts (axis values) of the arm 60 driven based on the command values.

The controller 31 displays an error on the touch panel 331, and receives an operation to reset the error by an operation on the touch panel 331. Thus, after the operator checks the current axis values of the joints 64 (43) and the comparison values displayed on the touch panel 331 and determines that it is possible to recover from the error, the operator can perform an operation to reset the error on the same first display.

The controller 31 performs a control to transition to a reset mode in which an error reset operation is performed, based on an operation on the touch panel 331. Thus, the operator can check the display on the touch panel 331, and then perform an operation on the touch panel 331 to perform work in the reset mode in which an error is reset.

For example, as shown in FIG. 6, when an error notification is being displayed on the touch panel 331, corners 331a, 331b, 331c, and 331d of the touch panel 331 are touched in a predetermined order to transition to the reset mode in which an error is reset.

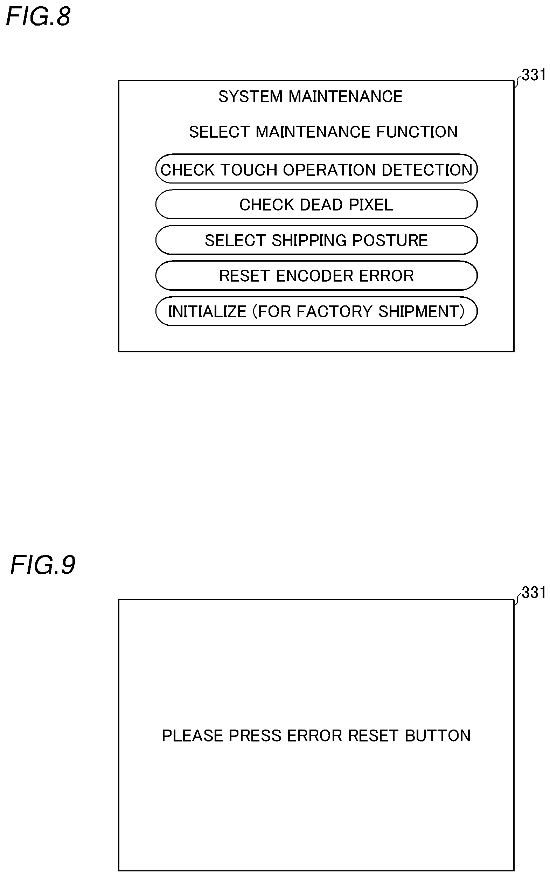

In the reset mode in which an error is reset, first, a password entry screen is displayed on the touch panel 331 as shown in FIG. 7. When the operator enters a password on this screen, a screen for selecting system maintenance is displayed on the touch panel 331 as shown in FIG. 8. The authentication of the operator is not limited to password entry, but the operator may be authenticated by entering an ID of the operator.

On the screen for selecting system maintenance, for example, "CHECK TOUCH OPERATION DETECTION", "CHECK DEAD PIXEL", "SELECT SHIPPING POSTURE", "RESET ENCODER ERROR", and "INITIALIZE (FOR FACTORY SHIPMENT)" can be selected, as shown in FIG. 8. In this case, an operation is performed to recover from the drive amount error, and thus "RESET ENCODER ERROR" is selected.

When "RESET ENCODER ERROR" is selected on the screen for selecting system maintenance on the touch panel 331, a screen is displayed prompting the operator to recover from the error, as shown in FIG. 9. The operator resets the error by following the instructions on this screen and pressing the reset button 333 (see FIG. 4) located in the vicinity of the touch panel 331.

In other words, after an error reset operation is performed on the touch panel 331, the controller 31 resets the error by receiving an operation on the reset button 333 provided separately from the touch panel 331. Thus, the error reset operation can be performed with the reset button 333 provided separately from the touch panel 331, and thus error reset due to an unintentional operation on the touch panel 331 can be reduced or prevented.

When an error has been reset for one arm 60, the error notification screen shown in FIG. 6 is displayed again when an error has occurred in the drive amount for another arm 60.

In other words, the controller 31 determines whether or not the differences between the current axis values of the joints 64 and the past axis values of the joints 64 or the command values for the past axis values are greater than the threshold for each of the plurality of arms 60, and provides a notification regarding the error for each of the plurality of arms 60. Thus, the operator can check that an error has occurred for each of the plurality of arms 60 individually, and thus the operator can easily determine whether or not it is possible to recover from the error for each of the plurality of arms 60.

The controller 31 causes both the touch panel 331 of the medical manipulator 1 and the touch panel 23 of the remote control apparatus 2 to display the error, and causes the touch panel 331 of the medical manipulator 1 to display the current axis values of the joints 64 (43) and the comparison values without causing the touch panel 23 of the remote control apparatus 2 to display the current axis values of the joints 64 (43) and the comparison values. Thus, only the occurrence of the error is displayed on the touch panel 23 of the remote control apparatus 2 arranged apart from the robot, and the occurrence of the error as well as the current axis values of the joints 64 (43) and the comparison values are displayed on the touch panel 331 of the medical manipulator 1 arranged at a position close to the robot, and thus at the position close to the robot, the operator can determine whether or not the robot can recover from the error while checking the current axis values of the joints 64 (43) and the comparison values.

In addition to the reset button 333 of the medical manipulator 1 for resetting the error, another button for resetting the error may be provided on the remote control apparatus 2.

### Advantages of This Embodiment

According to this embodiment, the following advantages are achieved.

According to this embodiment, as described above, the current axis values indicating the drive amount of the joints 64 (43) and the comparison values comparable to the current axis values of the joints 64 (43) are displayed on the touch panel 331. Accordingly, when an error occurs because the differences between the current axis values of the joints 64 (43) and the past axis values of the joints 64 (43) or the command values are greater than the threshold, the current axis values of the joints 64 (43) and the comparison values comparable to the current axis values of the joints 64 (43) are displayed on the touch panel 331, and thus the operator can easily check how much the current axis values of the joints 64 (43) differ from the past axis values of the joints 64 (43) or the command values. Consequently, when an error occurs in the drive amount of the robot, the operator can easily determine whether or not the robot can recover from the error.

### Modified Examples

The embodiment disclosed this time must be considered as illustrative in all points and not restrictive. The scope of the present disclosure is not shown by the above description of the embodiment but by the scope of claims for patent, and all modifications (modified examples) within the meaning and scope equivalent to the scope of claims for patent are further included.

For example, while the example in which when at the time of operation restart after operation interruption of the medical manipulator 1, the differences between the current axis values of the joints based on the detection of the encoders and the command values at the time of operation interruption (past) are greater than the threshold, a notification regarding an error is provided has been shown in the aforementioned embodiment, the present disclosure is not limited to this. Instead of the command values at the time of operation interruption (past), the axis values of the joints based on detection of the encoders at the time of operation interruption (past) may be used.

While the example in which four arms are provided has been shown in the aforementioned embodiment, the present disclosure is not limited to this. The number of arms may be any number as long as at least one arm is provided.

While the example in which each of the arms and the positioner includes a 7-axis articulated robot has been shown in the aforementioned embodiment, the present disclosure is not limited to this. For example, each of the arms and the positioner may include an articulated robot having an axis configuration (six axes or eight axes, for example) other than the 7-axis articulated robot.

While the example in which the medical manipulator includes the medical cart, the positioner, the arm base, the arms has been shown in the aforementioned embodiment, the present disclosure is not limited to this. For example, the medical manipulator may not include the medical cart, the positioner, or the arm base, but may include only the arms.

While an example of the robotic surgical system including the medical manipulator as the robot has been shown in the aforementioned embodiment, the present disclosure is not limited to this. The robot may be a robot for purposes other than surgical assistance. For example, the robot may be an industrial or commercial robot.

While the example in which when the differences between the current axis values of the joints based on detection of the encoders and the past command values are greater than the threshold, a notification regarding an error is provided has been shown in the aforementioned embodiment, the present disclosure is not limited to this. When differences between current detection values of the encoders of the drive axes and past detection values of the encoders of the drive axes are equal to or greater than a predetermined value and a specific malfunction has occurred in the encoders, the controller may provide a notification regarding the error and cause the display to display the current axis values of the drive axes and the comparison values comparable to the current axis values of the drive axes. Thus, when the differences between the current detection values of the encoders of the drive axes and the past detection values of the encoders of the drive axes are equal to or greater than the predetermined value and a specific malfunction has occurred in the encoders, the operator can check the displayed current axis values of the drive axes and the displayed comparison values, and thus the operator can easily perform recovery work.

In such a case, when the differences between the current detection values of the encoders of the drive axes and the past detection values of the encoders of the drive axes are equal to or greater than a predetermined value of one or more rotations, and a malfunction has occurred in which rotation data, which is information on the numbers of rotations of the encoders, is lost from memories, the controller may provide a notification regarding the error and cause the display to display the current axis values of the drive axes and the comparison values comparable to the current axis values of the drive axes. Specifically, when the differences between the current detection values of the encoders of the drive axes and the past detection values of the encoders of the drive axes are equal to or greater than the predetermined value of one or more rotations, and a malfunction has occurred in which the rotation data of the encoders is lost from the memories due to a drop in power, the controller may provide a notification regarding the error and cause the display to display the current axis values of the drive axes and the comparison values comparable to the current axis values of the drive axes.

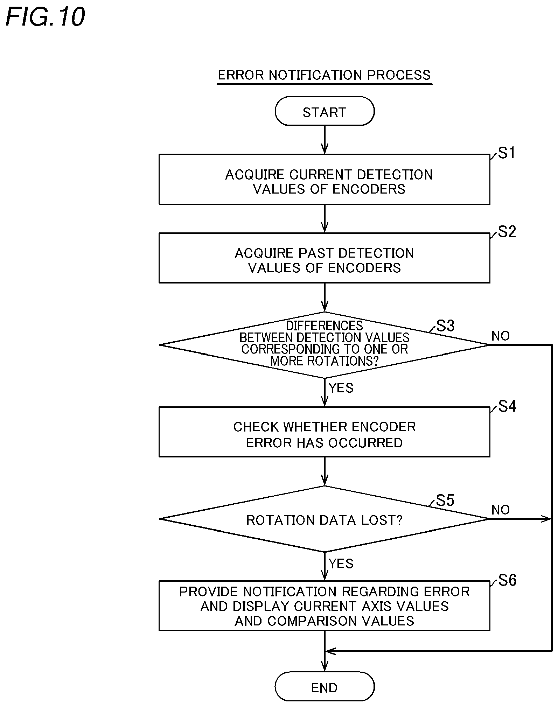

For example, the controller may perform a control such as an error notification process shown in FIG. 10.

In step S1 of FIG. 10, the controller acquires the current detection values of the encoders of the drive axes in step S1. In step S2, the controller acquires the past detection values of the encoders of the drive axes.

In step S3, the controller determines whether or not the differences between the current detection values of the encoders of the drive axes and the past detection values of the encoders of the drive axes correspond to one or more rotations. When the differences in the detection values correspond to one or more rotations, the process advances to step S4, and when the differences in the detection values correspond to less than one rotation, the error notification process is terminated. In step S4, the controller checks whether an encoder error has occurred. Specifically, the controller checks whether an error has occurred in which the rotation data of the encoders is lost from the memories due to a drop in power. The memories are provided in the encoders, and maintain the storage of the rotation data using power. In other words, when the power drops, the rotation data is lost from the memories.

In step S5, the controller determines whether or not the rotation data of the encoders has been lost from the memories. When the rotation data has been lost, the process advances to step S6. When the rotation data has not been lost, the error notification process is terminated. In step S6, the controller provides a notification regarding the error and causes the display to display the current axis values of the drive axes and the comparison values comparable to the current axis values of the drive axes.

The functionality of the elements disclosed herein may be implemented using circuitry or processing circuitry which includes general purpose processors, special purpose processors, integrated circuits, ASICs ("Application Specific Integrated Circuits"), conventional circuitry and/or combinations thereof which are configured or programmed to perform the disclosed functionality. Processors are considered processing circuitry or circuitry as they include transistors and other circuitry therein. The processor may be a programmed processor which executes a program stored in a memory. In the disclosure, the circuitry, units, or means are hardware that carry out or are programmed to perform the recited functionality. The hardware may be any hardware disclosed herein or otherwise known which is programmed or configured to carry out the recited functionality. When the hardware is a processor which may be considered a type of circuitry, the circuitry, means, or units are a combination of hardware and software, the software being used to configure the hardware and/or processor.

### Aspects

It will be appreciated by those skilled in the art that the exemplary embodiments described above are specific examples of the following aspects.

### (Item 1)

A robotic surgical system comprising:
a robot including a plurality of drive axes and an encoder on each of the plurality of drive axes to detect an axis value indicating a drive amount of the drive axis, the robot being operable to move a surgical instrument;
a controller; and
a first display; wherein
the controller is configured or programmed to, when a difference between a current axis value of the drive axis based on detection of the encoder and a past axis value of the drive axis based on detection of the encoder at a time at which the robot was stopped or a command value to drive the robot is greater than a threshold, provide a notification regarding an error and cause the first display to display the current axis value of the drive axis and a comparison value comparable to the current axis value of the drive axis.

### (Item 2)

The robotic surgical system according to item 1, wherein the controller is configured or programmed to cause the first display to display, as the comparison value, a value based on at least one of the past axis value of the drive axis, a difference value between the past axis value and the current axis value of the drive axis, the command value, a difference value between the command value and the current axis value, or the predetermined reference value.

### (Item 3)

The robotic surgical system according to item 1 or 2, wherein the controller is configured or programmed to, when a difference between the past axis value of the drive axis at a first time at which the robot was shut down or driving of the robot was interrupted and the current axis value of the drive axis at a second time at which the robot has been started up or the driving of the robot has been restarted after the first time is greater than the threshold, provide the notification regarding the error and cause the first display to display the current axis value of the drive axis at the second time and the comparison value based on at least one of the past axis value of the drive axis at the first time or the predetermined reference value.

### (Item 4)

The robotic surgical system according to any one of items 1 to 3, wherein the controller is configured or programmed to display the error on the first display and receive an operation to reset the error by an operation on the first display.

### (Item 5)

The robotic surgical system according to item 4, wherein the controller is configured or programmed to perform a control to transition to a reset mode in which the operation to reset the error is performed based on the operation on the first display.

### (Item 6)

The robotic surgical system according to item 4 or 5, wherein the controller is configured or programmed to receive an operation on a reset button provided separately from the first display after the operation to reset the error on the first display, and reset the error.

### (Item 7)

The robotic surgical system according to any one of items 1 to 6, wherein
the robot includes a plurality of robot arms; and
the controller is configured or programmed to determine whether or not the difference between the current axis value of the drive axis and the past axis value of the drive axis or the command value is greater than the threshold for each of the plurality of robot arms, and provide the notification regarding the error for each of the plurality of robot arms.

### (Item 8)

The robotic surgical system according to any one of items 1 to 7, comprising:
an operator apparatus including an operation unit to receive an operation to move the surgical instrument, and a second display; wherein
the controller is configured or programmed to cause both the first display and the second display to display the error, and cause the first display to display the current axis value of the drive axis and the comparison value without causing the second display to display the current axis value of the drive axis and the comparison value.

### (Item 9)

The robotic surgical system according to any one of items 1 to 8, wherein the controller is configured or programmed to, when a difference between a current detection value of the encoder of the drive axis and a past detection value of the encoder of the drive axis is equal to or greater than a predetermined value and a specific malfunction has occurred in the encoder, provide a notification regarding an error and cause the first display to display the current axis value of the drive axis and the comparison value comparable to the current axis value of the drive axis.

### (Item 10)

The robotic surgical system according to item 9, wherein the controller is configured or programmed to, when the difference between the current detection value of the encoder of the drive axis and the past detection value of the encoder of the drive axis is equal to or greater than a predetermined value of one or more rotations, and a malfunction has occurred in which rotation data, which is information on a number of rotations of the encoder, is lost from a memory, provide a notification regarding an error and cause the first display to display the current axis value of the drive axis and the comparison value comparable to the current axis value of the drive axis.

### (Item 11)

The robotic surgical system according to item 10, wherein the controller is configured or programmed to, when the difference between the current detection value of the encoder of the drive axis and the past detection value of the encoder of the drive axis is equal to or greater than the predetermined value of one or more rotations, and a malfunction has occurred in which the rotation data of the encoder is lost from the memory due to a drop in power, provide a notification regarding an error and cause the first display to display the current axis value of the drive axis and the comparison value comparable to the current axis value of the drive axis.

### (Item 12)

A surgical robot comprising:
a robot body including a plurality of drive axes and an encoder on each of the plurality of drive axes to detect an axis value indicating a drive amount of the drive axis, the robot body being operable to move a surgical instrument;
a controller; and
a display; wherein
the controller is configured or programmed to, when a difference between a current axis value of the drive axis based on detection of the encoder and a past axis value of the drive axis based on detection of the encoder at a time at which the robot body was stopped or a command value to drive the robot body is greater than a threshold, provide a notification regarding an error and cause the display to display the current axis value of the drive axis and a comparison value comparable to the current axis value of the drive axis.

### (Item 13)

The surgical robot according to item 12, wherein the controller is configured or programmed to, when a difference between a current detection value of the encoder of the drive axis and a past detection value of the encoder of the drive axis is equal to or greater than a predetermined value and a specific malfunction has occurred in the encoder, provide a notification regarding an error and cause the display to display the current axis value of the drive axis and the comparison value comparable to the current axis value of the drive axis.

### (Item 14)

A control method for a robotic surgical system, the robotic surgical system comprising a robot including a plurality of drive axes and an encoder on each of the plurality of drive axes to detect an axis value indicating a drive amount of the drive axis, the robot being operable to move a surgical instrument, the control method comprising:
when a difference between a current axis value of the drive axis based on detection of the encoder and a past axis value of the drive axis based on detection of the encoder at a time at which the robot was stopped or a command value to drive the robot is greater than a threshold, providing a notification regarding an error and causing a display to display the current axis value of the drive axis and a comparison value comparable to the current axis value of the drive axis.

### (Item 15)

The control method for the robotic surgical system according to item 14, comprising:
when a difference between a current detection value of the encoder of the drive axis and a past detection value of the encoder of the drive axis is equal to or greater than a predetermined value and a specific malfunction has occurred in the encoder, providing a notification regarding an error and causing the display to display the current axis value of the drive axis and the comparison value comparable to the current axis value of the drive axis.

### (Item 16)

A robot system comprising:
a robot including a plurality of drive axes and an encoder on each of the plurality of drive axes to detect an axis value indicating a drive amount of the drive axis;
a controller; and
a display; wherein
the controller is configured or programmed to, when a difference between a current axis value of the drive axis based on detection of the encoder and a past axis value of the drive axis based on detection of the encoder at a time at which the robot was stopped or a command value to drive the robot is greater than a threshold, provide a notification regarding an error and cause the display to display the current axis value of the drive axis and a comparison value comparable to the current axis value of the drive axis.

### (Item 17)

The robot system according to item 16, wherein the controller is configured or programmed to, when a difference between a current detection value of the encoder of the drive axis and a past detection value of the encoder of the drive axis is equal to or greater than a predetermined value and a specific malfunction has occurred in the encoder, provide a notification regarding an error and cause the display to display the current axis value of the drive axis and the comparison value comparable to the current axis value of the drive axis.

### Description of Reference Numerals

1: medical manipulator (surgical robot)
2: remote control apparatus (operator apparatus)
4: surgical instrument
21: operation manipulator arm (operation unit)
23: touch panel (second display)
31: controller
40: positioner (robot, robot body)
43: joint (drive axis)
50: arm base (robot, robot body)
60: arm (robot, robot arm, robot body)
64: joint (drive axis)
100: robotic surgical system (robot system)
331: touch panel (first display, display)
333: reset button
E1, E2, E3, E4: encoder

## Claims

1. A robotic surgical system comprising:
a robot including a plurality of drive axes and an encoder on each of the plurality of drive axes to detect an axis value indicating a drive amount of the drive axis, the robot being operable to move a surgical instrument;
a controller; and
a first display; wherein
the controller is configured or programmed to, when a difference between a current axis value of the drive axis based on detection of the encoder and a past axis value of the drive axis based on detection of the encoder at a time at which the robot was stopped or a command value to drive the robot is greater than a threshold, provide a notification regarding an error and cause the first display to display the current axis value of the drive axis and a comparison value comparable to the current axis value of the drive axis.

2. The robotic surgical system according to claim 1, wherein the controller is configured or programmed to cause the first display to display, as the comparison value, a value based on at least one of the past axis value of the drive axis, a difference value between the past axis value and the current axis value of the drive axis, the command value, a difference value between the command value and the current axis value, or the predetermined reference value.

3. The robotic surgical system according to claim 1, wherein the controller is configured or programmed to, when a difference between the past axis value of the drive axis at a first time at which the robot was shut down or driving of the robot was interrupted and the current axis value of the drive axis at a second time at which the robot has been started up or the driving of the robot has been restarted after the first time is greater than the threshold, provide the notification regarding the error and cause the first display to display the current axis value of the drive axis at the second time and the comparison value based on at least one of the past axis value of the drive axis at the first time or the predetermined reference value.

4. The robotic surgical system according to claim 1, wherein the controller is configured or programmed to display the error on the first display and receive an operation to reset the error by an operation on the first display.

5. The robotic surgical system according to claim 4, wherein the controller is configured or programmed to perform a control to transition to a reset mode in which the operation to reset the error is performed based on the operation on the first display.

6. The robotic surgical system according to claim 4, wherein the controller is configured or programmed to receive an operation on a reset button provided separately from the first display after the operation to reset the error on the first display, and reset the error.

7. The robotic surgical system according to claim 1, wherein
the robot includes a plurality of robot arms; and
the controller is configured or programmed to determine whether or not the difference between the current axis value of the drive axis and the past axis value of the drive axis or the command value is greater than the threshold for each of the plurality of robot arms, and provide the notification regarding the error for each of the plurality of robot arms.

8. The robotic surgical system according to claim 1, comprising:
an operator apparatus including an operation unit to receive an operation to move the surgical instrument, and a second display; wherein
the controller is configured or programmed to cause both the first display and the second display to display the error, and cause the first display to display the current axis value of the drive axis and the comparison value without causing the second display to display the current axis value of the drive axis and the comparison value.

9. The robotic surgical system according to claim 1, wherein the controller is configured or programmed to, when a difference between a current detection value of the encoder of the drive axis and a past detection value of the encoder of the drive axis is equal to or greater than a predetermined value and a specific malfunction has occurred in the encoder, provide a notification regarding an error and cause the first display to display the current axis value of the drive axis and the comparison value comparable to the current axis value of the drive axis.

10. The robotic surgical system according to claim 9, wherein the controller is configured or programmed to, when the difference between the current detection value of the encoder of the drive axis and the past detection value of the encoder of the drive axis is equal to or greater than a predetermined value of one or more rotations, and a malfunction has occurred in which rotation data, which is information on a number of rotations of the encoder, is lost from a memory, provide a notification regarding an error and cause the first display to display the current axis value of the drive axis and the comparison value comparable to the current axis value of the drive axis.

11. The robotic surgical system according to claim 10, wherein the controller is configured or programmed to, when the difference between the current detection value of the encoder of the drive axis and the past detection value of the encoder of the drive axis is equal to or greater than the predetermined value of one or more rotations, and a malfunction has occurred in which the rotation data of the encoder is lost from the memory due to a drop in power, provide a notification regarding an error and cause the first display to display the current axis value of the drive axis and the comparison value comparable to the current axis value of the drive axis.

12. A surgical robot comprising:
a robot body including a plurality of drive axes and an encoder on each of the plurality of drive axes to detect an axis value indicating a drive amount of the drive axis, the robot body being operable to move a surgical instrument;
a controller; and
a display; wherein
the controller is configured or programmed to, when a difference between a current axis value of the drive axis based on detection of the encoder and a past axis value of the drive axis based on detection of the encoder at a time at which the robot body was stopped or a command value to drive the robot body is greater than a threshold, provide a notification regarding an error and cause the display to display the current axis value of the drive axis and a comparison value comparable to the current axis value of the drive axis.

13. The surgical robot according to claim 12, wherein the controller is configured or programmed to, when a difference between a current detection value of the encoder of the drive axis and a past detection value of the encoder of the drive axis is equal to or greater than a predetermined value and a specific malfunction has occurred in the encoder, provide a notification regarding an error and cause the display to display the current axis value of the drive axis and the comparison value comparable to the current axis value of the drive axis.

14. A control method for a robotic surgical system, the robotic surgical system comprising a robot including a plurality of drive axes and an encoder on each of the plurality of drive axes to detect an axis value indicating a drive amount of the drive axis, the robot being operable to move a surgical instrument, the control method comprising:
when a difference between a current axis value of the drive axis based on detection of the encoder and a past axis value of the drive axis based on detection of the encoder at a time at which the robot was stopped or a command value to drive the robot is greater than a threshold, providing a notification regarding an error and causing a display to display the current axis value of the drive axis and a comparison value comparable to the current axis value of the drive axis.

15. The control method for the robotic surgical system according to claim 14, comprising:
when a difference between a current detection value of the encoder of the drive axis and a past detection value of the encoder of the drive axis is equal to or greater than a predetermined value and a specific malfunction has occurred in the encoder, providing a notification regarding an error and causing the display to display the current axis value of the drive axis and the comparison value comparable to the current axis value of the drive axis.

16. A robot system comprising:
a robot including a plurality of drive axes and an encoder on each of the plurality of drive axes to detect an axis value indicating a drive amount of the drive axis;
a controller; and
a display; wherein
the controller is configured or programmed to, when a difference between a current axis value of the drive axis based on detection of the encoder and a past axis value of the drive axis based on detection of the encoder at a time at which the robot was stopped or a command value to drive the robot is greater than a threshold, provide a notification regarding an error and cause the display to display the current axis value of the drive axis and a comparison value comparable to the current axis value of the drive axis.

17. The robot system according to claim 16, wherein the controller is configured or programmed to, when a difference between a current detection value of the encoder of the drive axis and a past detection value of the encoder of the drive axis is equal to or greater than a predetermined value and a specific malfunction has occurred in the encoder, provide a notification regarding an error and cause the display to display the current axis value of the drive axis and the comparison value comparable to the current axis value of the drive axis.
